# EUROPEAN PATENT APPLICATION

(11) **EP 3 311 806 A1**
(43) Date of publication of application: **25.04.2018**
(21) Application number: 17197299.5
(22) Date of filing: 19.10.2017
(51) Int. Cl.: A61K 31/047, A61K 31/164, A61P 31/00, A61P 43/00, A61P 15/02

(54) **COMPOSITION FOR REPAIR OF THE MUCOUS MEMBRANE**

(30) Priority: 21.10.2016 NL 2017656
(71) Applicant: Wagenaar, Louis Johan, 2314 GC Leiden (NL)
(72) Inventor: Wagenaar, Louis Johan, 2314 GC Leiden (NL)
(74) Representative: EP&C

(57) **Abstract**

Composition for use in the treatment of a mucous membrane condition wherein the composition comprises at least 2% by weight dexpanthenol and at least 8% by weight glycerol. The composition can be used orally to treat a condition of the mucous membrane of the mouth or gingival condition. The composition can also be used vaginally to treat a vaginal mucous membrane condition. The composition is particularly suitable to stimulate the secretion of fluids or mucus.

## Description

A dry or damaged mouth frequently appears to those who have undergone certain chemotherapies or radiation of their mouth and/or throat. Also people who have been taking high doses of morphine or any other medication with for instance an anticholinic (side)effect over a longer period of time suffer from this.

However, there is a range of other causes for long-lasting dryness of the mouth. So far a permanent cure has not been possible. In actual fact treatment has been rather inadequate: in, for example, hospice, hospital or during domiciliary care only some flavored ice (several times an hour) or a drink is offered which has hardly any or only an extremely short-term effect. Moreover, this treatment may even worsen the sensation of a dry mouth. Ice and sweet, savoury or bitter drinks can become repulsive or worsen the situation.

A dry mouth can be caused specifically by a lack of sufficient saliva. The salivary glands, for instance, produce too little saliva. An additional problem is that many patients suffer from small injuries on the mucous membrane, or even infections, often with fungi. In a dry mouth a small wound can easily tear and, in combination with a patient's low resistance, this could lead to an infection. Consequently, many patients have difficulties eating, but are also limited in their choice of food or hampered in speech. In addition, solid administering means, for instance of tablets, is therefore problematic.

A solution is not easy to be found. The causes have been looked into and they are rather wide-spread. Going through the literature and experience reports has revealed that a singular, univocal treatment is not easily defined.

When researching, the present inventor has first and foremost looked into botanic substances or mixtures thereof. Finding and identifying an acceptable botanic substance with the desired effects was not at all obvious. Preferentially a plant was to be found which could stimulate the required saliva production; better still a product that has a restraining influence on acetylcholinesterase. Best of all would be a substance that has neither side-effects nor application difficulties (such as the need to be stored in a cool environment, sensitivity towards fungi or special requirements with respect to its administering and the like), produces no discomfort, does not lead to boredom and that is effective for longer than just a few minutes. In short, quite a long list of requirements and preferences. The use of strong substances such as alkaloïds and cholinergics/cholinergica may be effective, but will also provide many undesirable side-effects.

Initially the area of dried plants was researched. These are, when chewed upon, able to stimulate saliva production. However, too many patients suffer from such a diminished production of saliva that little is to be expected from those. Many mouths have become raw or damaged by longlasting lack of sufficient saliva. This is why the inventor designed a gel which adheres to the mucous membrane which in itself already makes it longer-lasting due to the better exposure of the membrane to the substance. In the course of time a number of substances have been tested and added to the gel.

A number of substances prove to do well as an extra ingredient to the composition of the invention (according to claim 1 or 2): orange extract, peppermintoil and all sorts of botanical substances provide a great deal of relief for patients with a dry and/or damaged or infected mouth. A gel which attaches well to the mucous membrane is preferred over any other. A number of substances meet this requirement and therefore make them suitable as an extra ingredient of the composition of the invention (according to claim 1 and 2) such as, but not limited to, Carbopol 971 or 974, high viscoscious substances such as HPC or HPMC, whereas also other additions can be considered so as to ensure a longer-lasting impact on the mucous membrane such as carragene, gelatine, binding agents in all kinds of forms such as starches, polyvinylpyrrolidones or alginates as addition(s) to the composition of the invention (according to claim 1 or 2). All of the above in singular form as well as in mixtures, such as carragene and/or gelatine in singular form or in a variation such as PVP K25 with PVP K90 or cellulose(s) such as, but not limited to Klucel HF with Klucel GF may be added to the composition.

Besides, substances may be added to make the gel or fluid (further) stimulate the secretion of fluids such as the addition of glycerol or sorbitol. Sorbitol or glycerol can be used in a concentration of 0.1 to 50% w/w in a fluid or gel. The addition or replacement of components could be done to such an extent that the mass becomes so thick or solid that it can no longer be classified as a gel or fluid, but as a paste or even tablet or powder mixture instead.

The use of dexpanthenol in a mouthgel, mouthcream or mouth fluid is an advantage. In the invention, dexpanthenol can also be added as pantothenic acid (vitamin B5) or as salt or in another form. In the composition of the invention (according to claim 1 or 2) dexpanthenol or pantothenic acid or a salt thereof can be used in a concentration of 0.1 to 15% by weight.

In addition the application of the composition of the invention can be advantageous (according to claim 1 or 2):
- for the intestinal mucous membrane or in or around the anus
- after a course of chemotherapy or radiotherapy
- for the vaginal mucous membrane
- other mucous membranes.

Similar effects, but also additional or different effects can occur. An example would be the vagina, where repair, germ reduction or germ restraint can take place due to the application.

Damage to the mucous membrane can be reduced or even disappear completely due to the application.

Examples of different types of application of the composition of the invention (according to claim 1 or 2) are:
A. Mouthgel
   1. Sodium Pantothenate
   2. Water
   3. Carbopol 974 P
   4. Glycerol 85%
   5. Rosemary ethereal oil
B. Mouthrinse or drink
   1. Peppermint oil
   2. Sorbitol
   3. Water
   4. Klucel GF and Klucel HF
   5. Eucalyptus ethereal oil
C. Vaginal paste
   1. Glycerol
   2. Water
   3. Alginic acid and/or Xanthane
   4. Thyme ethereal oil
   5. Dexpanthenol
D. Mouthgel
   1. Dexpanthenol
   2. Water
   3. Carbopol 971 P
   4. Glycerol 100%
   5. Rosemary ethereal oil
   6. Thyme ethereal oil

Through the application of this composition with, amongst others, dexpanthenol (according to claim 1 or 2) this invention offers possibilities for the reduction or temporarily or complete disappearance of a dry mouth. It can also be used to relieve damaged mouths, aphthas or infections or other affections in the mouth such as, but not limited to, small cracks in the mucous membrane or bleeding gums.

### Examples

A study with more than 50 cancer patients has revealed that the composition of the invention with dexpanthenol, glycerol and possibly one or more ethereal oils was capable to make the dryness of the mouth disappear completely immediately after each application for some considerable time - at least half an hour or much longer. One third of this group of cancer patients also suffered from mouth infections, especially Candida, because the resistance of these patients is particularly low. These infections and damage already virtually disappeared within a short time, usually within just a few days. Such an appreciable effect which, in addition, was also instantly noticeable, came as an utter surprise to those conducting the research.

### Toothpaste and gums

Even after a relatively short-term application of the composition of the invention with dexpanthenol and glycerol (according to claim 1 or 2) irritated or affected gums improved quickly, especially when used as a gel or paste. Large quantitites are not necessary; the application of one or just a few millilitres during a few treatments proved to be sufficient to be effective without any side-effects.

### Vagina

Meanwhile it has appeared that injuries to the mucous membrane of the vagina the composition mentioned above can be successfully applied. A patient (48) who had experienced the strong effect of the composition used as a gel in her mouth and who also suffered from a fungal infection in her vagina used the gel also around and in her vagina. To everybody's surprise the fungal infection quickly receeded after only a few applications. The application of the composition of the invention with dexpanthenol and glycerol (preferably as a gel) resulted in a flush of fungal residue and the patients soon felt a lot better. This effect was completely unexpected.

### Other types of application

It is advantageous that the composition is non-perishable outside a cooler or refrigerator and remains intact at room temperature. An additional advantage is that several variations are available, especially for mouth applications, for example by adding one or more flavours or ethereal oils.

An added advantage is that for he majority of the applications no artificial preservation additives, such as parabenes, sorbates or others need to be included.

The application of the composition as mentioned above (according to claim 1 or 2) can, even by the sole application of dexpanthenol in combination with ethereal oils or possibly even without these oils, even be effective in the treatment of foot fungus, fungal nails, or application in the anus when undergoing chemotherapy. The use of dexpanthenol as gel or spray or roller as part of a deodorant is also useful and sensible.

Whenever dexpanthenol and glycerol are used (together) a combination of at least 1 % w/w dexpanthenol and at least 5% w/w pure glycerol is advised although 3% w/w dexpanthenol or more and 10% w/w glycerol or more is preferred. If the system for which it is designed can manage it 45% w/w glycerol or more is advised. The same applies to 5% w/w of dexpanthenol or more. The intended system of dexpanthenol and glycerol is preferably not to be applied to the eyes.

If required medicinal substances can be added tot the composition, for instance by dissolving them in glycerol or the glycerol-water mixture. Propyleneglycol lends itself particularly for the dissolution of medicinal substances which are more difficult to be absorbed in glycerol or the water system without further intervention such as solubilisation or the use of emulsifiers. When desired a desinfectant effect can be enhanced by (partly) applying propyleneglycol in the composition of the invention. Application of 5-25% w/w propyleneglycol is the most sensible. Wherever glycerol is mentioned in the description or claim this can be substituted by glycerol and/or propyleneglycol.

A number of medicines have the side-effect of making the mucous membranes reduce their production of saliva. These are mainly medicines with an anti-cholinergetic (side-)effect used for the treatment of, amongst others, high blood pressure (anti-hypertensives), arrhythmia, or medicines such as anti-depressants, sleeping tablets, diuretics or morphine. These medicines usually do not themselves affect the mucous membranes, but do reduce the production of saliva. The composition of the present invention (according to claim 1 or 2) could very effectively be used in combination with such medicines.

The composition (according to claim 1 or 2) can potentially be free of dexpanthenol or free of glycerol. As an alternative the composition can contain a maximum of 90, 80, 70, 60, 50, 40, 30, 20, 10 or 5% w/w of dexpanthenol and/or a maximum of 90, 80, 70, 60, 50, 40, 30, 20, 15 or 10% w/w glycerol. To supplement or as further alternative for the composition (according to claim 1 or 2) it can contain less dexpanthenol/glycerol, for example at least 1 to 1.5% w/w dexpanthenol and/or at least 1, 2, 3, 4, 5, 6, 7% w/w glycerol. Instead of the application during the treatment of a mucous membrane condition or the moisturisation of a mucous membrane the composition (according to claim 1) can also be used:
- in the treatment of an infection, for example a microbial infection or fungal infection;
- in the treatment of a dry mouth (xerostomy);
- in the treatment of (internal) injuries;
- for its anti-mycotic or germ reduction properties;
- other applications.

The composition (according to claim 1 or 2) can be in the form of a gel or a paste and have the viscosity of at least 20,000, 30,000, 40,000, 50,000, 60,000, 70,000, 80,000, 90,000 or 100,000mPa.s. The viscosity can be measured with a Brookfeld RV viscometer at room temperature (20 degrees Celsius) at the appropriate rotation and the appropriate spindle and after 1 minute. The composition may include water as basic component, such as at least 10, 20, 30, 40, 50, 60, 70, 80, 90, 95% w/w.

## Claims

1. Composition for use in the treatment of a dry mouth wherein the composition comprises at least 2% by weight dexpanthenol and at least 8% by weight glycerol.

2. Composition for use in the treatment of a condition, wherein the composition comprises at least 2% by weight dexpanthenol and at least 8% by weight glycerol and wherein the composition is for stimulating fluid secretion or mucus secretion.

3. Composition according to any one of claim 1 or 2 wherein the composition comprises at least 2.5, 3, 4, 5, 6, 7, 8, 9, 10, 20, 30, 40, 50, 60, 70, 80, 90 or 99% by weight dexpanthenol.

4. Composition according to any one of claims 1-3 wherein the composition comprises at least 8,5 , 9, 10, 11, 12, 13, 14, 15, 16, 17, 18, 19, 20, 30, 40, 50, 60, 70, 80, 90 or 95% by weight glycerol.

5. Composition according to any one of claims 1-4 wherein the composition is for oral application.

6. Composition according to any one of claims 2-4 wherein the condition is a vaginal mucous membrane condition and/or for wherein the composition is for vaginal application.

7. Composition according to any of the claims 1-4 wherein the composition is for the treatment of an infection, preferably a microbial or fungal infection.

8. Composition according to any one of claims 1-7 wherein the composition also comprises one or more ethereal oils.

9. Composition according to any one of claims 1-8 in the form of a toothpaste, cream, rinsing fluid or gel.

10. Composition according to any one of claims 1-9 further comprising a sticking and/or thickening substance, preferably chosen from HPMC, HPC, carbomere, starch or a starch derivative, polyvinyl(derivative), cellulose or a celloluse derivative, gelatine and alginate.
